# EUROPÄISCHE PATENTSCHRIFT

(11) **EP 0 678 242 B1**
(45) Veröffentlichungstag und Bekanntmachung des Hinweises auf die Patenterteilung: **07.04.1999**
(21) Anmeldenummer: 94119662.8
(22) Anmeldetag: 13.12.1994
(51) Int. Cl.: A22C 5/00, B08B 3/00, B08B 3/08, A61L 2/06, A61L 2/24

(54) **Verfahren zur Reinigung von Arbeitsgeräten für die Lebensmittelverarbeitung**
Process for cleaning food processing machine
Procédé de nettoyage d'une machine de traitement des aliments

(30) Priorität: 15.12.1993 DE 4342674
(43) Veröffentlichungstag der Anmeldung: 25.10.1995
(73) Patentinhaber: FREUND Maschinenfabrik GmbH & Co. KG, 33100 Paderborn (DE)
(72) Erfinder: Seroka, Karl-Heinz, Dipl.-Ing., D-33154 Salzkotten (DE); Syré, Manfred, Dipl.-Ing., D-33100 Paderborn (DE); Freund, Robert, Dipl.-Ing. Dipl.-Wirt.-Ing., D-33100 Paderborn (DE)
(74) Vertreter: Stracke, Alexander, Dipl.-Ing.

(56) Entgegenhaltungen:
- DE-A- 2 712 020
- DE-A- 3 209 305
- FR-A- 2 565 854
- US-A- 3 561 040
- US-A- 4 688 585
- US-A- 5 265 628

## Beschreibung

Die Erfindung betrifft ein Verfahren gemäß dem Oberbegriff des Anspruches 1.

Die strengen hygienischen Anforderungen bei der Verarbeitung von Lebensmitteln, insbesondere von Fleisch, fordern von den betroffenen Verarbeitungsbetrieben eine ständige, turnusmäßige Reinigung und Desinfektion der von den Mitarbeitern eingesetzten Arbeitsgeräte. Da für die Zeitdauer der Reinigungs- und Desinfektionsvorgänge die üblichen Arbeitsvorgänge unterbrochen werden müssen, wirkt sich eine Verkürzung der Reinigungszyklen direkt proportional auf eine Produktivitätssteigerung aus. Aus diesem Grunde werden vielfach die vorgeschriebenen und notwendigen Reinigungs- und Desinfektionsmaßnahmen der Arbeitsgeräte unzulässig verkürzt oder überhaupt nicht wahrgenommen. Ungenügend oder gar nicht gereinigte Arbeitsgeräte können dann zu Kontaminationen von bearbeiteten Fleischoberflächen führen.

Aus der DE 27 12 020 A1 ist eine Dekontaminationsanlage für chirurgische Instrumente und dergleichen bekannt, bei dem die zu behandelnden Instrumente zur Dekontamination mehrere Behandlungsstufen durchlaufen. Jede Behandlungsstufe erfolgt innerhalb einer individuell auf den Behandlungsvorgang abgestimmten Baueinheit. Diese Baueinheit beinhaltet eine automatische Innenfördereinrichtung für das zu behandelnde Instrument sowie eine automatische Schiebetür an der Ein- und Auslaßseite. Der Betriebsablauf wird durch eine automatische Steuereinrichtung gesteuert. Dies betrifft insbesondere die Innenfördereinrichtung und die Schiebetüren.

Diese vorbekannte Dekontaminationsanlage ist für die Reinigung von Arbeitsgeräten mit angetriebenen, beweglichen Bauteilen für die Lebensmittelverarbeitung nicht geeignet. Die Steuerung kann so manipuliert werden, daß die vorgeschriebenen Reinigungszyklen nicht eingehalten werden. Außerdem ist es notwendig, daß das Arbeitsgerät stets in gleicher Lage dem Desinfektionszyklus unterworfen wird, da nur bestimmte Bauteile mit den Lebensmitteln, beispielsweise Fleisch, in Berührung kommen. Dies sind beispielsweise Sägen, das Sägeblatt oder das Sägeband.

Ausgehend von einem durch die DE 27 12 020 A1 vorbekannten Stand der Technik, liegt der Erfindung die Aufgabe zugrunde, ein Verfahren zur Reinigung von Arbeitsgeräten für die Lebensmittelverarbeitung so zu gestalten, daß eine unzulässige Manipulation der vorgeschriebenen Reinigungs- und Desinfektionsintervalle zuverlässig ausgeschlossen wird und daß die Dauer der Reinigungs- und Desinfektionsintervalle auf das absolut notwendige Maß beschränkt wird.

Die Aufgabe wird erfindungsgemäß gelöst, indem das nach Ablauf des Arbeitszyklus abgeschaltete Arbeitsgerät in einer definierten Endlage innerhalb der Reinigungseinrichtung festgelegt und daß durch die definierte Endlage der kontrollierte Desinfektionszyklus ausgelöst wird.

Zunächst wird nach Ablauf eines Arbeitszyklus vom Bediener normalerweise das Arbeitsgerät abgeschaltet. Dies bewirkt nach Ablauf einer bestimmten, vorwählbaren und vom Bedienungspersonal nicht beeinflußbaren Zeit, daß das Arbeitsgerät nicht mehr betriebsbereit ist und trotz Betätigung des Einschalters nicht mehr anläuft. Ein erneutes Einschalten des Arbeitsgerätes ist nach der Abschaltung nur möglich, wenn der Desinfektionszyklus vollständig durchgeführt wurde.

In weiterer Ausgestaltung ist vorgesehen, daß eine Zwangssteuerung zuverlässig ausschließt, daß der notwendige Desinfektionszyklus nicht wahrgenonmmen wird oder der begonnene Vorgang vorzeitig durch Herausnahme des Arbeitsgerätes aus der Reinigungseinrichtung abgebrochen wird.

Zur Durchführung des Desinfektionszyklus wird das Arbeitsgerät vom Bediener in die Reinigungseinrichtung eingeführt und darin innerhalb einer definierten Endlage in einer Halterung verriegelt. Die Verriegelung wird durch die Zwangssteuerung überwacht. Dadurch wird bewirkt, daß ein Entnehmen des Arbeitsgerätes vor Ende des Desinfektionszyklus nicht möglich ist und daß zum anderen sich nach der Verriegelung das Arbeitsgerät in der für den optimalen Reinigungsvorgang am besten geeigneten Position befindet. Ist das Arbeitsgerät verriegelt, so wird durch die Zwangssteuerung der Zuführschlitz zur Einführung des Arbeitsgerätes geschlossen und es wird durch Einschalten eines optischen und/oder akustischen Signals der Beginn des Desinfektionszyklus angezeigt.

Es hat sich als besonders vorteilhaft erwiesen, den Desinfektionszyklus in drei einzelne Arbeitsschritte aufzugliedern. Zunächst wird das Arbeitsgerät mit Reinigungsfluid, das durch in der Reinigungseinrichtung angeordnete Sprühdüsen austritt, abgespült und von schmutz- und eiweißhaltigen Anhaftungen befreit. Gleichzeitig mit der Außenreinigung wird durch die Zwangssteuerung bewirkt, daß aus üblicherweise im Inneren des Arbeitsgerätegehäuses angeordneten Reinigungsdüsen ebenfalls Reinigungsfluid austritt, um das Arbeitsgerät auch innen von Anhaftungen zu befreien. Dieser Innenreinigungsprozeß wird zusätzlich dadurch unterstützt, daß die Zwangssteuerung das in der Halterung festgelegte Arbeitsgerät selbsttätig einschaltet, vorausgesetzt, es wurde vom Bediener auch abgeschaltet, damit dann auch eventuell innerhalb des Gehäuses gekapselte Maschinenteile abgesprüht werden. Das selbsttätige Anlaufen des Arbeitsgerätes durch die Zwangssteuerung ist zwangsgekoppelt mit der Festlegung des Arbeitsgerätes, so daß der eventuelle Versuch des Bedienungspersonals während des gesamten Desinfektionszyklus das vermeintlich wieder betriebsbereite Arbeitsgerät zu entnehmen, wirkungslos bleiben muß. Die Dauer des Abspülvorganges ist durch eine Zeiteinstellung vorwählbar, kann jedoch durch das Bedienungspersonal nicht beeinflußt werden. Nach Ende der Reinigung mit kaltem Reinigungsfluid erfolgt die Desinfektion des Arbeitsgerätes mit einem geeigneten Desinfektionsmittel, vorzugsweise mit heißem Wasser. Bei diesem Arbeitsschritt werden die innerhalb der Reinigungseinrichtung und innerhalb des Arbeitsgerätes vorhandenen Sprühdüsen mit dem Desinfektionsmittel beaufschlagt, wobei gleichzeitig, wie bereits oben beim Reinigungsvorgang beschrieben, das Arbeitsgerät in der Aufnahmehalterung in Betrieb gesetzt wird. Die Dauer des Desinfektionsschrittes ist wiederum einstellbar aber durch das Bedienpersonal nicht beeinflußbar.

Ist der Desinfektionsschritt beendet, werden die Sprühdüsen wiederum mit Kaltwasser beaufschlagt, um das Arbeitsgerät auf Normaltemperatur abzukühlen. Gleichzeitig wird durch den Kühlwassersprühvorgang das Kondensieren der nach Beendigung des Desinfektionsvorganges noch vorhandenen Dampfschwaden im Gehäuse der Reinigungseinrichtung unterstützt. Der durch das Kaltwasser bewirkte Abkühlzyklus kann ebenso wie der vorangegangene Reinigungs- und Desinfektionszyklus in zeitlicher Länge vorgewählt werden.

Die Aufteilung des Desinfektionszyklus in drei Arbeitsschritte hat insbesondere den Vorteil, daß durch die Reinigung mit Reinigungsfluid Eiweißcoagulationen zuverlässig verhindert werden und der nachfolgende Desinfektionsschritt bei einem gereinigten Arbeitsgerät naturgemäß kürzer ausfallen kann als bei einem nur ungenügend gereinigten, noch mit Anhaftungen versehenen Arbeitsgerät. Der den Reinigungs- und Desinfektionsschriten nachgeschaltete Abkühlvorgang verhindert das Auftreten von mechanischen Problemen, beispielsweise dem Lösen von Lagern innerhalb der Maschine, erhöht somit die Lebensdauer des eingesetzten Arbeitsgerätes und erleichtert außerdem die Handhabung durch das Bedienungspersonal, da Verletzungen durch überhitzte Maschinenteile ausgeschlossen sind.

Nach Ende des Desinfektionszyklus schaltet die Zwangssteuerung das eingeschaltete optische und/oder akustische Überwachungssignal wieder aus und entriegelt das Arbeitsgerät innerhalb der Halterung. Das Bedienpersonal kann nunmehr das Arbeitsgerät aus der Reinigungseinrichtung entnehmen, wobei die Zwangssteuerung die elektrische Außerbetriebsetzung des Arbeitsgerätes aufhebt, so daß dem Bedienungspersonal für den nächsten Arbeitszyklus ein funktionsfähiges, desinfiziertes Arbeitsgerät zur Verfügung steht.

Ist das Arbeitsgerät aus der Reinigungseinrichtung entnommen worden, so bewirkt die Zwangssteuerung den Anlauf des Selbstreinigungsvorganges der Reinigungseinrichtung. Sämtliche Innenflächen einschließlich der Abdichtungen des Zuführschlitzes werden durch aus speziellen Reinigungssprühdüsen austretendes Reinigungsfluid abgespült und gereinigt. Hierbei ist gewährleistet, daß dem Bedienungspersonal für jeden Reinigungs- und Desinfektionsvorgang eines Arbeitsgerätes eine saubere Reinigungseinrichtung zur Verfügung steht.

## Patentansprüche

1. Verfahren zur Reinigung von Arbeitsgeräten für die Lebensmittelverarbeitung, bei dem der Desinfektionszyklus des Arbeitsgerätes in einer sich nach der Entnahme des Arbeitsgerätes selbst reinigenden Reinigungsvorrichtung durchgeführt wird, **dadurch gekennzeichnet, daß** das nach Ablauf des Arbeitszyklus abgeschaltete Arbeitsgerät in einer definierten Endlage innerhalb der Reinigungseinrichtung festgelegt und durch die definierte Endlage der kontrollierte Desinfektionszyklus ausgelöst wird.

2. Verfahren nach Anspruch 1, **dadurch gekennzeichnet, daß** der Desinfektionszyklus von einer Zwangssteuerung kontrolliert wird.

3. Verfahren nach Anspruch 1, **dadurch gekennzeichnet, daß** das Arbeitsgerät durch eine Verriegelung in der Endlage festgelegt wird.

4. Verfahren nach Anspruch 1, **dadurch gekennzeichnet, daß** der Desinfektionszyklus aus einer Abfolge von drei Arbeitsschritten besteht.

5. Verfahren nach Anspruch 4, **dadurch gekennzeichnet, daß** der Desinfektionszyklus einen Reinigungsschritt, einen Desinfektionsschritt und einen Kühlschritt beinhaltet.

6. Verfahren nach Anspruch 3, **dadurch gekennzeichnet, daß** das Arbeitsgerät nach Ablauf des Desinfektionszyklus entriegelt wird.

7. Verfahren nach einem oder mehreren der vorhergehenden Ansprüche, **dadurch gekennzeichnet, daß** der Reinigungsvorgang durch optische und/oder akustische Mittel angezeigt wird.

8. Verfahren nach einem oder mehreren der vorhergehenden Ansprüche, **dadurch gekennzeichnet, daß** die Zwangssteuerung den Einführ- und Entnahmevorgang sowie ein Verschließen und Öffnen der Reinigungseinrichtung überwacht.

9. Verfahren nach Anspruch 1, **dadurch gekennzeichnet, daß** während des Selbstreinigungsvorganges alle Begrenzungsflächen des Innenraumes der Reinigungseinrichtung einschließlich vorhandener Abdichtungen von Zuführöffnungen der Reinigungsvorrichtung abgespült werden.

## Claims

1. A method of cleaning working devices for foodstuffs processing in which the disinfecting cycle of the working device is implemented in a cleaning apparatus which cleans itself after removal of the working device characterised in that the working device which is switched off after termination of the working cycle is fixed in a defined end position within the cleaning apparatus and the controlled disinfecting cycle is triggered off by the defined end position.

2. A method according to claim 1 characterised in that the disinfecting cycle is controlled by a positive control.

3. A method according to claim 1 characterised in that the working device is fixed in the end position by a locking means.

4. A method according to claim 1 characterised in that the disinfecting cycle comprises a sequence of three working steps.

5. A method according to claim 4 characterised in that the disinfecting cycle includes a cleaning step, a disinfecting step and a cooling step.

6. A method according to claim 3 characterised in that the working device is unlocked after termination of the disinfecting cycle.

7. A method according to one or more of the preceding claims characterised in that the cleaning operation is indicated by optical and/or acoustic means.

8. A method according to one or more of the preceding claims characterised in that the positive control monitors the introduction and removal procedure as well as opening and closing of the cleaning apparatus.

9. A method according to claim 1 chacterised in that during the self-cleaning procedure all surfaces defining the internal space of the cleaning apparatus including seals provided at feed openings of the cleaning apparatus are rinsed off.

## Revendications

1. Procédé de nettoyage d'appareils de traitement de produits alimentaires, dans lequel le cycle de désinfection de l'appareil est mis en oeuvre dans un dispositif de nettoyage qui se nettoie tout seul une fois retiré l'appareil, caractérisé en ce qu'une fois terminé le cycle de travail, l'appareil mis hors circuit est bloqué dans une position terminale définie, à l'intérieur du dispositif de nettoyage, et en ce que le cycle de désinfection contrôlé est déclenché par la position terminale définie.

2. Procédé selon la revendication 1, caractérisé en ce que le cycle de désinfection est contrôlé par une commande forcée.

3. Procédé selon la revendication 1, caractérisé en ce que l'appareil est bloqué dans la position terminale par un système de verrouillage.

4. Procédé selon la revendication 1, caractérisé en ce que le cycle de désinfection est constitué d'une succession de trois étapes.

5. Procédé selon la revendication 4, caractérisé en ce que le cycle de désinfection comprend une étape de nettoyage, une étape de désinfection et une étape de refroidissement.

6. Procédé selon la revendication 3, caractérisé en ce que l'appareil est déverrouillé une fois achevé le cycle de désinfection.

7. Procédé selon l'une ou plusieurs des revendications précédentes, caractérisé en ce que l'étape de nettoyage est indiquée par des moyens optiques et/ou acoustiques.

8. Procédé selon l'une ou plusieurs des revendications précédentes, caractérisé en ce que la commande forcée contrôle le processus d'introduction et de retrait, ainsi qu'une fermeture et une ouverture du dispositif de nettoyage.

9. Procédé selon la revendication 1, caractérisé en ce qu'au cours du processus d'auto-nettoyage, toutes les surfaces qui délimitent l'espace intérieur du dispositif de nettoyage, y compris les joints des ouvertures d'introduction du dispositif de nettoyage, sont lavées.
